# EUROPEAN PATENT APPLICATION

(11) **EP 4 360 535 A1**
(43) Date of publication of application: **01.05.2024**
(21) Application number: 22837753.7
(22) Date of filing: 07.07.2022
(51) Int. Cl.: A61B 3/10

(54) **IMAGE PROCESSING METHOD, IMAGE PROCESSING PROGRAM, IMAGE PROCESSING DEVICE, AND OPHTHALMIC DEVICE**

(30) Priority: 07.07.2021 JP 2021112899
(71) Applicant: NIKON CORPORATION, Minato-ku Tokyo 108-6290 (JP)
(72) Inventor: TANABE, Yasushi, Tokyo 108-6290 (JP); FUJIWARA, Tomoharu, Tokyo 108-6290 (JP); TOYAMA, Motoki, Tokyo 108-6290 (JP); MUKAI, Mariko, Tokyo 108-6290 (JP)
(74) Representative: Hoffmann Eitle
(86) International application number: PCT/JP2022/027008
(87) International publication number: WO 2023/282339

(57) **Abstract**

Image processing performed by a processor and including acquiring a first fundus image of an examined eye, acquiring a position for acquiring a tomographic image of the examined eye's fundus, acquiring a second fundus image of the examined eye, determining the acquired position is included in a specific range of the first fundus image, and computing a first movement amount of the examined eye in a case in which the acquired position is included in the specific range, using first registration processing to positionally align the first fundus image and the second fundus image, and of computing a second movement amount of the examined eye in a case in which the acquired position falls outside the specific range, using second registration processing to positionally align the first fundus image and the second fundus image, the second registration processing being different from the first registration processing.

## Description

### Technical Field

Technology disclosed herein relates to an image processing method and program, an image processing device, and an ophthalmic device.

### Background Art

A tracking method to move an optical system to match movement of an examined eye is disclosed in the specification of US Patent Application Laid-Open No. 2019/0059723. Imaging of a fundus image without blurring has hitherto been demanded.

### SUMMARY OF INVENTION

An image processing method of a first aspect of technology disclosed herein is image processing performed by a processor. The image processing includes a step of acquiring a first fundus image of an examined eye, a step of acquiring a position for acquiring a tomographic image of the examined eye's fundus, which is set using the first fundus image, a step of acquiring a second fundus image of the examined eye, a step of determining whether or not the acquired position is included in a specific range of the first fundus image, and a step of computing a first movement amount of the examined eye in a case in which the acquired position is included in the specific range, using first registration processing to positionally align the first fundus image and the second fundus image, and of computing a second movement amount of the examined eye in a case in which the acquired position falls outside the specific range, using second registration processing to positionally align the first fundus image and the second fundus image, the second registration processing being different from the first registration processing.

An image processing device of a second aspect of technology disclosed herein is an image processing device including a processor. The processor executes image processing including a step of acquiring a first fundus image of an examined eye, a step of acquiring a position for acquiring a tomographic image of the examined eye's fundus, which is set using the first fundus image, a step of acquiring a second fundus image of the examined eye,a step of determining whether or not the acquired position is included in a specific range of the first fundus image, and a step of computing a first movement amount of the examined eye in a case in which the acquired position is included in the specific range, using first registration processing to positionally align the first fundus image and the second fundus image, and of computing a second movement amount of the examined eye in a case in which the acquired position falls outside the specific range, using second registration processing to positionally align the first fundus image and the second fundus image, the second registration processing being different from the first registration processing.

A non-transitory computer-readable medium storing an image processing program of a third aspect of technology disclosed herein causes a computer to execute image processing. The image processing includes a step of acquiring a first fundus image of an examined eye, a step of acquiring a position for acquiring a tomographic image of the examined eye's fundus, which is set using the first fundus image, a step of acquiring a second fundus image of the examined eye,a step of determining whether or not the acquired position is included in a specific range of the first fundus image, and a step of computing a first movement amount of the examined eye in a case in which the acquired position is included in the specific range, using first registration processing to positionally align the first fundus image and the second fundus image, and of computing a second movement amount of the examined eye in a case in which the acquired position falls outside the specific range, using second registration processing to positionally align the first fundus image and the second fundus image, the second registration processing being different from the first registration processing.

### BRIEF DESCRIPTION OF DRAWINGS

Fig. 1 is a block diagram of an ophthalmic system 100.
Fig. 2 is a schematic configuration diagram illustrating an overall configuration of an ophthalmic device 110.
Fig. 3 is a block diagram of functions of a CPU 16A of a control device 16 of the ophthalmic device 110.
Fig. 4 is a flowchart illustrating a program executed by the CPU 16A of the ophthalmic device 110.
Fig. 5 is a flowchart illustrating a subroutine of eye-tracking processing of step 306 of Fig. 4.
Fig. 6 is a diagram illustrating a fundus center region and a fundus peripheral region of an eyeball.
Fig. 7A is a diagram illustrating a UWF-SLO fundus image 400G.
Fig. 7B is a diagram illustrating the UWF-SLO fundus image 400G with a position 402 for acquiring OCT data superimposed thereon.
Fig. 7C is a diagram illustrating the UWF-SLO fundus image 400G with the position 402 for acquiring OCT data and a region 400 for acquiring a rectangular SLO fundus image superimposed thereon.
Fig. 8 is a diagram illustrating a screen 500 of a display of a viewer 150.

### DESCRIPTION OF EMBODIMENTS

Detailed explanation follows regarding embodiments of the present disclosure, with reference to the drawings.

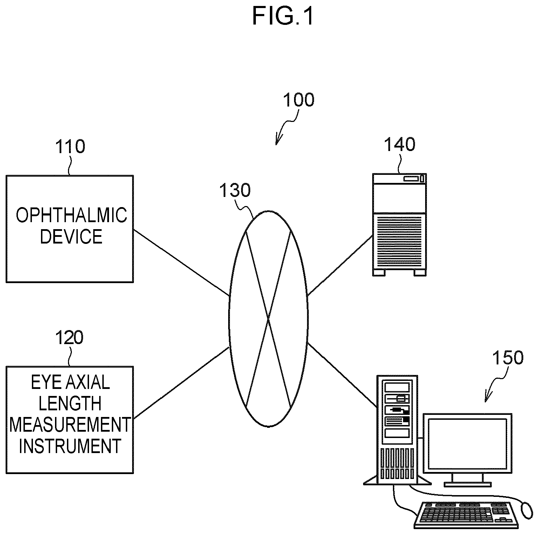

Explanation follows regarding a configuration of an ophthalmic system 100, with reference to Fig. 1. As illustrated in Fig. 1, the ophthalmic system 100 includes an ophthalmic device 110, an eye axial length measurement instrument 120, a management server device (referred to hereafter as "server") 140, and an image display device (referred to hereafter as "viewer") 150. The ophthalmic device 110 acquires a fundus image. The eye axial length measurement instrument 120 measures the axial length of an eye of an examinee. The server 140 stores fundus images that were obtained by imaging the fundus of examinees using the ophthalmic device 110 in association with IDs of the examinees. The viewer 150 displays medical information such as fundus images acquired from the server 140.

The ophthalmic device 110 is an example of an "image processing device" of technology disclosed herein.

The ophthalmic device 110, the eye axial length measurement instrument 120, the server 140, and the viewer 150 are connected together through a network 130. The network 130 is a freely selected network such as a LAN, WAN, the Internet, or a wide area Ethernet. For example, a LAN may be employed as the network 130 when the ophthalmic system 100 is built in a single hospital.

Note that another ophthalmic instrument (an examination instrument such as for field of view measurement or ophthalmotonometery) or a diagnostic assistance device to perform image analysis using artificial intelligence may be connected to the ophthalmic device 110, the eye axial length measurement instrument 120, the server 140, and the viewer 150 through the network 130.

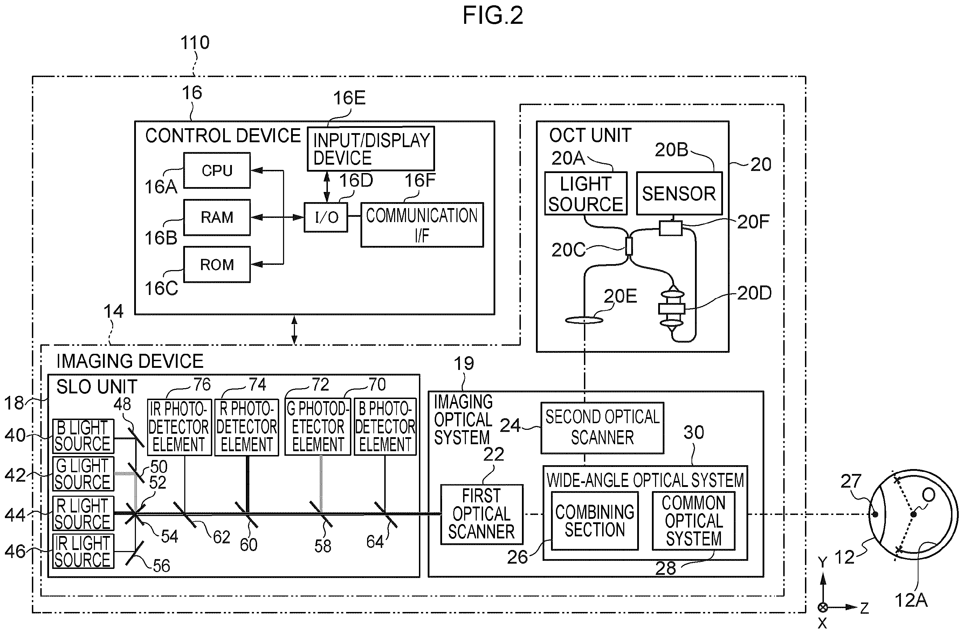

Next, explanation follows regarding a configuration of the ophthalmic device 110, with reference to Fig. 2.

For ease of explanation, scanning laser ophthalmoscope is abbreviated to SLO. Optical coherence tomography is also abbreviated to OCT.

With the ophthalmic device 110 installed on a horizontal plane and a horizontal direction taken as an X direction, a direction perpendicular to the horizontal plane is denoted a Y direction, and a direction connecting the center of the pupil at the anterior eye portion of the examined eye 12 and the center of the eyeball is denoted a Z direction. The X direction, the Y direction, and the Z direction are thus mutually perpendicular directions.

The ophthalmic device 110 includes an imaging device 14 and a control device 16. The imaging device 14 is provided with an SLO unit 18, an OCT unit 20, and an imaging optical system 19, and acquires a fundus image of the fundus of the examined eye 12. Two-dimensional fundus images that have been acquired by the SLO unit 18 are referred to hereafter as SLO fundus images. Tomographic images, front images of fundus (en-face images), and the like of the retina created based on OCT data acquired by the OCT unit 20 are referred to hereafter as OCT images.

The control device 16 includes a computer provided with a Central Processing Unit (CPU) 16A, Random Access Memory (RAM) 16B, Read-Only Memory (ROM) 16C, and an input/output (I/O) port 16D. An image processing program, described later, is stored in the ROM 16C. Note that control device 16 may be further provided with an external storage device and store the image processing program on the external storage device.

The image processing program is an example of a "program" of technology disclosed herein. The ROM 16C (or the external storage device) is an example of "memory" or "computer-readable storage medium" of technology disclosed herein. The CPU 16A is an example of a "processor" of technology disclosed herein. The control device 16 is an example of a "computer program product" of technology disclosed herein.

The control device 16 is provided with an input/display device 16E connected to the CPU 16A through the I/O port 16D. The input/display device 16E includes a graphical user interface to display images of the examined eye 12 and to receive various instructions from a user. An example of the graphical user interface is a touch panel display.

The control device 16 is also provided with a communication interface (I/F) 16F connected to the I/O port 16D. The ophthalmic device 110 is connected to the eye axial length measurement instrument 120, the server 140, and the viewer 150 through the communication interface (I/F) 16F and the network 130.

Although the control device 16 of the ophthalmic device 110 is provided with the input/display device 16E as illustrated in Fig. 2 and described above, the technology disclosed herein is not limited thereto. For example, a configuration may adopted in which the control device 16 of the ophthalmic device 110 is not provided with the input/display device 16E, and instead a separate input/display device physically independent of the ophthalmic device 110 is provided. In such cases, the display device is provided with an image processing processor unit that operates under the control of the CPU 16A in the control device 16. Such an image processing processor unit may be configured so as to display SLO fundus images, OCT images, and the like based on an image signal output as an instruction by the CPU 16A.

The imaging device 14 operates under the control of the CPU 16A of the control device 16. The imaging device 14 includes the SLO unit 18, the imaging optical system 19, and the OCT unit 20. The imaging optical system 19 includes a first optical scanner 22, a second optical scanner 24, and a wide-angle optical system 30.

The first optical scanner 22 scans light emitted from the SLO unit 18 two dimensionally in the X direction and the Y direction. The second optical scanner 24 scans light emitted from the OCT unit 20 two dimensionally in the X direction and the Y direction. As long as the first optical scanner 22 and the second optical scanner 24 are optical elements capable of deflecting light beams, they may be configured by any out of, for example, polygon mirrors, mirror galvanometers, or the like. A combination thereof may also be employed.

The wide-angle optical system 30 includes an objective optical system (not illustrated in Fig. 2) provided with a common optical system 28, and a combining section 26 that combines light from the SLO unit 18 with light from the OCT unit 20.

The objective optical system of the common optical system 28 may be a reflection optical system employing a concave mirror such as an elliptical mirror, a refraction optical system employing a wide-angle lens, or may be a reflection-refraction optical system employing a combination of a concave mirror and a lens. Employing a wide-angle optical system that utilizes an elliptical mirror, wide-angle lens, or the like enables imaging to be performed not only of a central portion of the fundus where the optic nerve head and macular are present, but also of the retina at a peripheral portion of the fundus where an equatorial portion of the eyeball and vortex veins are present.

Observation of the fundus over a wide field of view (FOV) 12A is implemented by employing the wide-angle optical system 30. The FOV 12A refers to a range capable of being imaged by the imaging device 14. The FOV 12A may be expressed as a viewing angle. In the present exemplary embodiment the viewing angle may be defined in terms of an internal illumination angle and an external illumination angle. The external illumination angle is the angle of illumination by a light beam illuminated from the ophthalmic device 110 toward the examined eye 12, and is an angle of illumination defined with respect to a pupil 27. The internal illumination angle is the angle of illumination of a light beam illuminated onto the fundus, and is an angle of illumination defined with respect to an eyeball center O. A correspondence relationship exists between the external illumination angle and the internal illumination angle. For example, an external illumination angle of 120° is equivalent to an internal illumination angle of approximately 160°. The internal illumination angle in the present exemplary embodiment is 200°.

SLO fundus images obtained by imaging at an imaging angle having an internal illumination angle of 160° or greater are referred to as UWF-SLO fundus images. UWF is an abbreviation of ultra-wide field.

An SLO system is implemented by the control device 16, the SLO unit 18, and the imaging optical system 19 as illustrated in Fig. 2. The SLO system is provided with the wide-angle optical system 30, enabling fundus imaging over the wide FOV 12A.

The SLO unit 18 is provided with plural light sources such as, for example, a blue (B) light source 40, a green (G) light source 42, a red (R) light source 44, an infrared (for example near infrared (IR)) light source 46, and optical systems 48, 50, 52, 54, 56 to guide the light from the light sources 40, 42, 44, 46 onto a single optical path using reflection or transmission. The optical systems 48, 50, 56 are configured by mirrors, and the optical systems 52, 54 are configured by beam splitters. B light is reflected by the optical system 48, transmitted through the optical system 50, and reflected by the optical system 54. G light reflected by the optical systems 50, 54, R light transmitted through the optical systems 52, 54, and IR light reflected by the optical systems 56, 52 are thereby respectively guided onto a single optical path.

The SLO unit 18 is configured so as to be capable of switching between the light source or the combination of light sources employed for emitting laser light of different wavelengths, such as a mode in which G light, R light and B light are emitted, a mode in which infrared light is emitted, etc. Although the example in Fig. 2 includes four light sources, i.e. the B light (blue light) source 40, the G light source 42, the R light source 44, and the IR light source 46, the technology disclosed herein is not limited thereto. For example, the SLO unit 18 may, furthermore, also include a white light source, in a configuration in which light is emitted in various modes, such as a mode in which white light is emitted alone.

Light introduced to the imaging optical system 19 from the SLO unit 18 is scanned in the X direction and the Y direction by the first optical scanner 22. The scanning light passes through the wide-angle optical system 30 and the pupil 27 and is illuminated onto the posterior eye portion of the examined eye 12. Reflected light that has been reflected by the fundus passes through the wide-angle optical system 30 and the first optical scanner 22 and is introduced into the SLO unit 18.

The SLO unit 18 is provided with a beam splitter 64 that, from out of the light coming from the posterior eye portion (e.g. fundus) of the examined eye 12, reflects the B light therein and transmits light other than B light therein, and a beam splitter 58 that, from out of the light transmitted by the beam splitter 64, reflects the G light therein and transmits light other than G light therein. The SLO unit 18 is further provided with a beam splitter 60 that, from out of the light transmitted through the beam splitter 58, reflects R light therein and transmits light other than R light therein. The SLO unit 18 is further provided with a beam splitter 62 that reflects IR light from out of the light transmitted through the beam splitter 60.

The SLO unit 18 is provided with plural photodetector elements corresponding to the plural light sources. The SLO unit 18 includes a B photodetector element 70 for detecting B light reflected by the beam splitter 64, and a G photodetector element 72 for detecting G light reflected by the beam splitter 58. The SLO unit 18 also includes an R photodetector element 74 for detecting R light reflected by the beam splitter 60 and an IR photodetector element 76 for detecting IR light reflected by the beam splitter 62.

Light that has passed through the wide-angle optical system 30 and the first optical scanner 22 and been introduced into the SLO unit 18 (i.e. reflected light that has been reflected by the fundus) is reflected by the beam splitter 64 and photo-detected by the B photodetector element 70 when B light, and is transmitted through the beam splitter 64 and reflected by the beam splitter 58 and photo-detected by the G photodetector element 72 when G light. When the incident light is R light, this light is transmitted through the beam splitters 64, 58, reflected by the beam splitter 60, and photo-detected by the R photodetector element 74. When the incident light is IR light, this light is transmitted through the beam splitters 64, 58, 60, reflected by the beam splitter 62, and photo-detected by the IR photodetector element 76. The CPU 16A employs signals detected by the B photodetector element 70, the G photodetector element 72, the R photodetector element 74, and the IR photodetector element 76 to generate UWF-SLO fundus images.

The UWF-SLO fundus image (sometimes referred to as a UWF fundus image or an original fundus image as described later) encompasses a UWF-SLO fundus image obtained by imaging the fundus in green (a green fundus image), and a UWF-SLO fundus image obtained by imaging the fundus in red (a red fundus image). The UWF-SLO fundus image further encompasses a UWF-SLO fundus image obtained by imaging the fundus in blue (a blue fundus image), and a UWF-SLO fundus image obtained by imaging the fundus in IR (an IR fundus image).

The control device 16 also controls the light sources 40, 42, 44 so as to emit light at the same time. A green fundus image, a red fundus image, and a blue fundus image are obtained with mutually corresponding positions by imaging the fundus of the examined eye 12 at the same time with the B light, G light, and R light. An RGB color fundus image is obtained from the green fundus image, the red fundus image, and the blue fundus image. The control device 16 obtains a green fundus image and a red fundus image with mutually corresponding positions by controlling the light sources 42, 44 so as to emit light at the same time and by imaging the fundus of the examined eye 12 at the same time with the G light and R light. An RG color fundus image is obtained from the green fundus image and the red fundus image.

Specific examples of the UWF-SLO fundus image accordingly include a blue fundus image, a green fundus image, a red fundus image, an IR fundus image, an RGB color fundus image, and an RG color fundus image. The image data for the respective UWF-SLO fundus images are transmitted from the ophthalmic device 110 to the server 140 through the communication interface (I/F) 16F, together with examinee information input through the input/display device 16E. The image data of the respective UWF-SLO fundus images and the examinee information are stored associated with each other in a storage device 254. The examinee information includes, for example, examinee ID, name, age, visual acuity, a right eye/left eye discriminator, and the like. The examinee information is input by an operator through the input/display device 16E.

An OCT system is implemented by the control device 16, the OCT unit 20, and the imaging optical system 19 illustrated in Fig. 2. The OCT system is provided with the wide-angle optical system 30. This enables fundus imaging to be performed over the wide FOV 12A similarly to when imaging the SLO fundus images as described above. The OCT unit 20 includes a light source 20A, a sensor (detector element) 20B, a first light coupler 20C, a reference optical system 20D, a collimator lens 20E, and a second light coupler 20F.

Light emitted from the light source 20A is split by the first light coupler 20C. After one part of the split light has been collimated by the collimator lens 20E into parallel light to serve as measurement light, the parallel light is introduced into the imaging optical system 19. The measurement light is scanned in the X direction and the Y direction by the second optical scanner 24. The scanning light is illuminated onto the fundus through the wide-angle optical system 30 and the pupil 27. Measurement light that has been reflected by the fundus passes through the wide-angle optical system 30 and the second optical scanner 24 so as to be introduced into the OCT unit 20. The measurement light then passes through the collimator lens 20E and the first light coupler 20C before being incident to the second light coupler 20F.

The other part of the light emitted from the light source 20A and split by the first light coupler 20C is introduced into the reference optical system 20D as reference light, and is made incident to the second light coupler 20F through the reference optical system 20D.

The respective lights that are incident to the second light coupler 20F, namely the measurement light reflected by the fundus and the reference light, interfere with each other in the second light coupler 20F so as to generate interference light. The interference light is photo-detected by the sensor 20B. The CPU 16A performs signal processing such as Fourier transformation on a detection signal detected by the sensor 20B and generates OCT data. The CPU 16A generates OCT images, such as tomographic images and en-face images, based on this OCT data.

The OCT system is able to acquire OCT data of an imaging area implemented by the wide-angle optical system 30.

The OCT data, tomographic images, and en-face images generated by the CPU 16A are transmitted, together with information about the examinee, from the ophthalmic device 110 to the server 140 via the communication interface (I/F) 16F. The OCT data and each of the various OCT images such as tomographic images and en-face images are associated with the examinee information and stored in the storage device 254.

Note that although in the present exemplary embodiment an example is given in which the light source 20A is a swept-source OCT (SS-OCT), the light source 20A may be configured from various types of OCT system, such as a spectral-domain OCT (SD-OCT) or a time-domain OCT (TD-OCT) system.

Next, description follows regarding the eye axial length measurement instrument 120. The eye axial length measurement instrument 120 measures an eye axial length that is a length in an eye axial direction of the examined eye 12.

The eye axial length measurement instrument 120 transmits the measured eye axial length to the server 140. The server 140 stores the eye axial length of the examinee associated with an examinee ID.

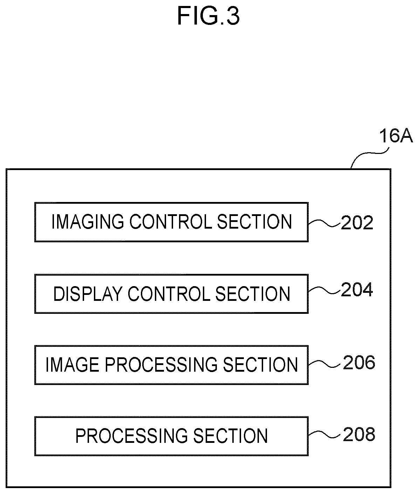

Next, description follows regarding each function implemented by the CPU 16A of the ophthalmic device 110 executing an ophthalmic instrument control program, with reference to Fig. 3. The ophthalmic instrument control program includes an imaging control function, a display control function, an image processing function, and a processing function. The CPU 16A functions as an imaging control section 202, a display control section 204, an image processing section 206, and a processing section 208 as illustrated in Fig. 3 by the CPU 16A executing the ophthalmic instrument control program including each of these functions.

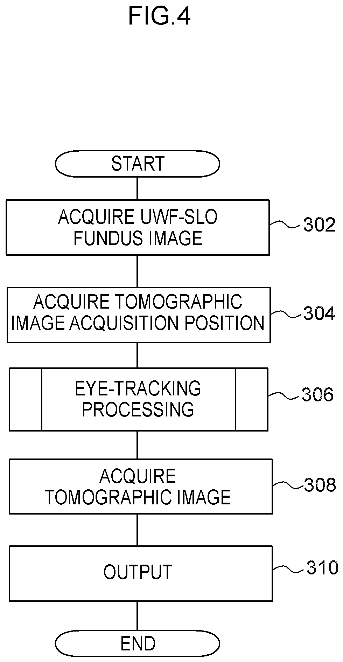

Detailed explanation now follows regarding image processing of the ophthalmic device 110, with reference to Fig. 4. Ophthalmic instrument control is implemented as illustrated in the flowchart of Fig. 4 by the CPU 16A of the control device 16 of the ophthalmic device 110 executing the ophthalmic instrument image processing program.

The processing illustrated in the flowchart of Fig. 4 is an example of a "image processing method" of technology disclosed herein.

An operator of the ophthalmic device 110 gets the examinee to place their chin on a non-illustrated support section of the ophthalmic device 110, and adjusts the position of the examined eye 12 of the examinee.

The display control section 204 of the ophthalmic device 110 displays a menu screen for input of examinee information and for mode selection on a screen of the input/display device 16E. The mode is for example an SLO mode to acquire SLO fundus images or an OCT mode to acquire OCT fundus images. The CPU 16A starts execution of the ophthalmic instrument control program illustrated in Fig. 4 when the operator has input the information about the examinee through the input/display device 16E and selected the OCT mode.

At step 302, the imaging control section 202 controls the SLO unit 18 and the imaging optical system 19, and acquires a first fundus image of the fundus of the examined eye 12, specifically UWF-SLO fundus images at three wavelength types by causing light to be emitted by the B light source 40, the G light source 42, and the R light source 44. Note that as described above, the UWF-SLO fundus images include a green fundus image, a red fundus image, a blue fundus image, and an RGB color fundus image.

The UWF-SLO fundus images are examples of a "first fundus image" of technology disclosed herein.

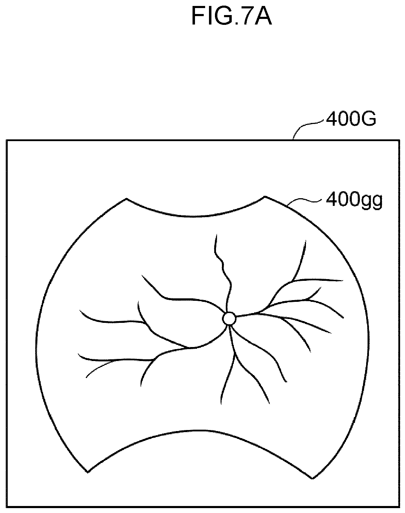

At step 304, the display control section 204 displays a UWF-SLO fundus image 400G on the display of the input/display device 16E. An UWF-SLO fundus image 400G displayed on the display is illustrated in Fig. 7A. The UWF-SLO fundus image 400G corresponds to an image of a region scannable with the SLO unit 18 and, as illustrated in Fig. 7A, includes a fundus region 400gg of the examined eye formed by guiding reflected light from the examined eye 12 fundus itself.

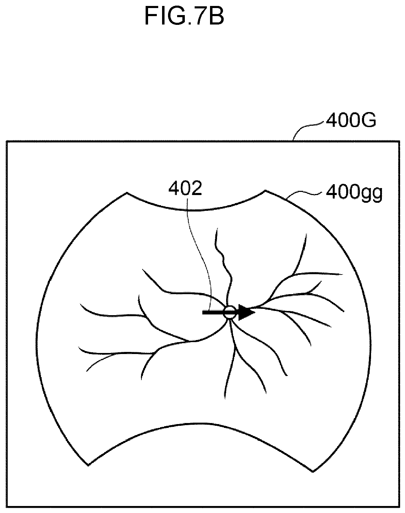

A user (operator of the ophthalmic device 110) uses the UWF-SLO fundus image 400G being displayed to set a region for acquiring OCT data (position for acquiring a tomographic image) using a touch panel and a non-illustrated input device. Fig. 7B illustrates a case in which the UWF-SLO fundus image 400G is employed to set the position 402 for acquiring a tomographic image as a straight line along the X direction. When the position 402 for acquiring the tomographic image has been set as the straight line, the position 402 for acquiring the tomographic image is displayed with an arrow.

Note that the position 402 for acquiring the tomographic image is not limited to being the straight line along the X direction as illustrated in Fig. 7B and may be a given single point or line, such as for example a straight line along the Y direction, a straight line intersecting with both the X direction and the Y direction, a curve connecting two points, or may be a given plane such as, for example, a circular region, a rectangular region, or the like.

In cases in which the position for acquiring the tomographic image is a single point, OCT data (called "A-scan data") is obtained by scanning in a depth (optical axis) direction at the single point on the fundus.

In cases in which the position for acquiring the tomographic image is a line, OCT data (called "B-scan data") is obtained by performing an A-scan plural times while moving the A-scan along this line (called a "B-scan").

In cases in which the position for acquiring the tomographic image is a plane, OCT data (called "C-scan data") is obtained by repeatedly performing a B-scan while moving the B-scan along this plane (called a "C-scan"). Three dimensional OCT data is generated by such a C-scan, and a two dimensional en-face image or the like is generated based on this three dimensional OCT data.

The processing section 208 then acquires position data (coordinate data or the like) of the position 402 for acquiring the tomographic image as set using the UWF-SLO fundus image 400G. The data illustrating the position to acquire the tomographic image is not limited to being coordinate data and may be a number or the like to roughly identify a position of the fundus image.

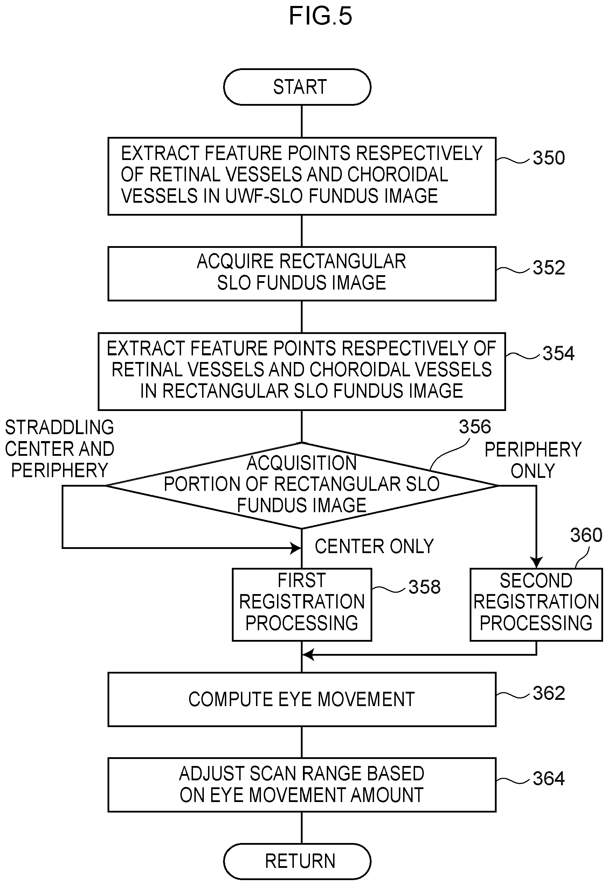

Eye-tracking processing is executed at step 306. Description follows regarding eye-tracking processing, with reference to Fig. 5.

Note that the technology disclosed herein is not limited to executing the eye-tracking processing immediately when the position data of the position 402 for acquiring the tomographic image is acquired as set using the UWF-SLO fundus image 400G at step 304. The operator, for example, confirms that alignment between the examined eye 12 of the examinee and the ophthalmic device 110 is in an appropriate state. The operator instructs start of OCT imaging when confirmed that an appropriate state of alignment has been achieved by operating a button or the like on the display of the input/display device 16E. The eye-tracking processing is accordingly executed when an operation to start OCT imaging has been instructed in this manner.

At step 350 of Fig. 5, the image processing section 206 uses a red fundus image and a green fundus image of the UWF-SLO fundus images to extract feature points respectively in the retinal vessels and choroidal vessels. More specifically, the image processing section 206 first extracts each of the retinal vessels and choroidal vessels, and extracts feature points respectively in the retinal vessels and choroidal vessels.

First, description follows regarding a method to respectively extract the retinal vessels and choroidal vessels using the red fundus image and the green fundus image.

The structure of an eye is one in which a vitreous body is covered by plural layers of differing structure. The plural layers include, from the vitreous body at the extreme inside to the outside, the retina, the choroid, and the sclera. R light passes through the retina and reaches the choroid. The red fundus image therefore includes information relating to blood vessels present in the retina (retinal vessels) and information relating to blood vessels present in the choroid (choroidal vessels). In contrast thereto, G light only reaches as far as the retina. The green fundus image accordingly only includes information relating to the blood vessels present in the retina (retinal vessels).

The image processing section 206 extracts retinal vessels from the green fundus image by executing image processing such as black hat filter processing on the green fundus image. A retinal vascular image resulting from extracting only pixels of the retinal vessels from the green fundus image is obtained thereby. Black hat filter processing is processing to find a difference between image data of the green fundus image that is a source image, and image data obtained by closing processing in which dilation processing is performed N times (wherein N is an integer of 1 or more) on this source image followed by erosion processing being performed N times thereon.

Next, the image processing section 206 removes the retinal vessels by performing inpainting processing or the like using the retinal vascular image extracted from the red fundus image and from the green fundus image. The inpainting processing is processing to set the pixel values of a specific position such that a difference to a mean value of surrounding pixels is within a specific range (for example, zero). Namely, infill processing is performed using the position information of the retinal vessels extracted from the green fundus image to infill pixels corresponding to the retinal vessels of the red fundus image with the same values as surrounding pixels. A choroidal vascular image is thereby obtained from the red fundus image resulting from extracting only the pixels of the retinal vessels. Furthermore, the image processing section 206 may also perform emphasis processing on the red fundus image from which the retinal vessels have been removed by performing contrast limited adaptive histogram equalization (CLAHE) processing thereon to emphasize the choroidal vessels.

Next, description follows regarding a method to extract feature points respectively in the retinal vessels and choroidal vessels using the red fundus image and the green fundus image.

The image processing section 206 extracts branch points or merge points of the retinal vessels from the retinal vascular image as first feature points of the retinal vessels. Branch points or merge points of the choroidal vessels are then extracted from the choroidal vascular image as first feature points of the choroidal vessels. These retinal vessel first feature points and choroidal vessel first feature points are then stored by the processing section 208 in the RAM 16B.

At step 352, the imaging control section 202 controls the SLO unit 18 and the imaging optical system 19 so as to acquire a rectangular SLO fundus image of the fundus of the examined eye 12. Note that the rectangular SLO fundus image of the fundus of the examined eye 12 is an example of a "second fundus image" of the technology disclosed herein.

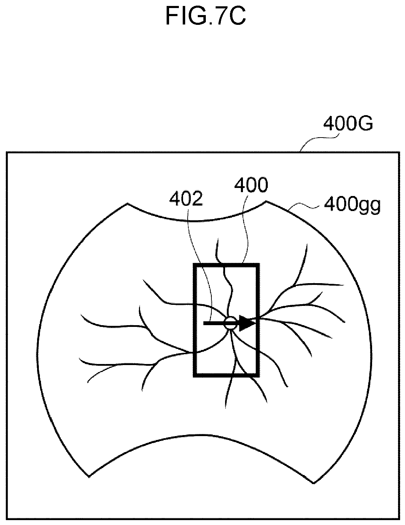

More specifically, first the operator uses the UWF-SLO fundus image 400G to set the region for acquiring the rectangular SLO fundus image. Fig. 7C illustrates the UWF-SLO fundus image 400G having the region 400 for acquiring the rectangular SLO fundus image superimposed thereon, as well as the position 402 for acquiring a tomographic image. In the example illustrated in Fig. 7C, the region 400 for acquiring the rectangular SLO fundus image is a region including all of the position 402 for acquiring a tomographic image.

The technology disclosed herein is not limited to cases in which the region 400 for acquiring the rectangular SLO fundus image is a region containing all of the position 402 for acquiring the tomographic image. For example, the region for acquiring the rectangular SLO fundus image may include only part of the position 402 for acquiring the tomographic image. The region for acquiring the rectangular SLO fundus image may be configured so as to include at least part of the position 402 for acquiring the tomographic images.

The region for acquiring the rectangular SLO fundus image may be configured so as not to include the position 402 for acquiring the tomographic images. The region for acquiring the rectangular SLO fundus image may accordingly be set irrespective of the position 402 for acquiring the tomographic images.

Note that cases in which the region 400 for acquiring the rectangular SLO fundus image is a region including all of the position 402 for acquiring the tomographic images enable an increase in the chance of narrowing a search range to calculate a movement amount of the eye, described later, compared to cases in which the region 400 does not include any of the position 402 or includes only part thereof. This thereby enables eye-tracking processing to be performed smoothly, namely enables processing time to be shortened.

The region for acquiring the rectangular SLO fundus image includes at least part of the fundus region 400gg of the examined eye.

The size of the region for acquiring the rectangular SLO fundus image is smaller than the size of the UWF-SLO fundus image 400G. Namely, the angle of view of the rectangular SLO fundus image is an angle of view smaller than the imaging angle of view of the UWF-SLO fundus images and is, for example, set as an angle of view of from 10° to 50°.

At step 352, the imaging control section 202 acquires the position of the region 400 for acquiring the rectangular SLO fundus image as set using the UWF-SLO fundus image 400G. The processing section 208 stores and retains the position of the region for acquiring the rectangular SLO fundus image as a first position in the RAM 16B. The imaging control section 202 controls the SLO unit 18 and the imaging optical system 19 based on the position of this acquired region 400, and acquires the rectangular SLO fundus image of the fundus of the examined eye 12 by acquiring an image of the fundus of the examined eye 12. Note that when acquiring the rectangular SLO fundus image, the imaging control section 202 images the fundus by causing the IR light source 46 to emit light so as to illuminate IR light onto the fundus. This approach is adopted in order not make the examinee feel blinded, due to IR light not being sensed by photoreceptor cells of the retina of the examined eye.

Next at step 354, the image processing section 206 extracts the feature points respectively of the retinal vessels and choroidal vessels in the rectangular SLO fundus image of the second fundus image.

In the rectangular SLO fundus image imaged using the IR light, the retinal vessels appear as narrow black blood vessels and the choroidal vessels appear as thick white blood vessels. The image processing section 206 performs black hat processing on the rectangular SLO fundus image to identify the pixel positions of the retinal vessels. Second feature points of the retinal vessels are thereby obtained.

Next, the image processing section 206 removes the retinal vessels from the rectangular SLO fundus image by performing inpainting processing at the pixel positions of the retinal vessels, and extract the second feature points of the choroidal vessels from the rectangular SLO fundus image from which the retinal vessels have been removed. The processing section 208 stores the second feature points of the choroidal vessels on the RAM 16B.

Next, at step 356 the image processing section 206 determines whether or not the position 402 for acquiring the tomographic image is contained in a specific range, falls outside this specific range, or part thereof is contained in this specific range.

The specific range is, for example, a center region of the fundus. In such cases the image processing section 206 determines, based on data indicating the position 402 for acquiring the tomographic image, whether or not the position 402 for acquiring the tomographic image is contained in the fundus center region, contained in a peripheral region and not contained in the center region, or straddles both the center region and the peripheral region.

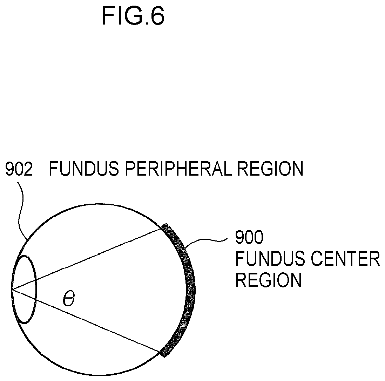

A center region 900 of the fundus is, as illustrated in Fig. 6, a circular region having a specific radius centered on a point where the optical axis of the ophthalmic device 110 passes through the eyeball center and intersects with the fundus. A region of processing surface area outside the center region 900 of the fundus is a fundus peripheral region 902.

This specific range is not limited to being the center region 900 of the fundus. For example, the specific range may be a circular region having a specific radius centered on a macular portion, and may be a predetermined range where the blood vessel density of the retinal vessels is a specific value or greater.

The image processing (in particular eye-tracking processing) proceeds to step 358 in cases in which the position 402 for acquiring the tomographic image is included in the fundus center region 900, and cases in which the position 402 for acquiring the tomographic image straddles the fundus center region 900 and the fundus peripheral region 902. The image processing (in particular eye-tracking processing) proceeds to step 360 in cases in which the position 402 for acquiring the tomographic image is positioned in the fundus peripheral region 902 alone.

At step 358, the image processing section 206 executes first registration processing having processing content optimized for the fundus center region so as to achieve a comparatively short processing time and facilitate image matching. At step 360, the image processing section 206 executes second registration processing having processing content optimized for the peripheral region so as to facilitate image matching even though the processing time is comparatively long.

The registration processing is processing to positionally align the rectangular SLO fundus image with the UWF-SLO fundus images (in particular the RGB color fundus image). More specifically, this is processing by image matching to identify at which position to position the rectangular SLO fundus image on the RGB color fundus image.

In the image matching of the first registration processing at step 358, the image processing section 206 extracts, as feature points, three individual second feature points of the retinal vessels extracted from the rectangular SLO fundus image. The image processing section 206 searches for a position where these three second feature points are aligned with the first feature points of the retinal vessels as extracted from the RGB color fundus image. The processing section 208 stores and retains the position matched in the first registration processing as a second position in the RAM 16B.

Note that de-noise processing is not performed on the RGB color fundus image in the first registration processing of step 358.

In the image matching of the second registration processing of step 360, the image processing section 206 first performs de-noise processing on the RGB color fundus image. The image processing section 206 then extracts, as feature points, 3 individual feature points from both the second feature points of the retinal vessels and the second feature points of the choroidal vessels extracted from the rectangular SLO fundus image, a total of 6 individual feature points. The image processing section 206 then searches for a position where these 6 feature points are aligned with the first feature points of the retinal vessels or the first feature points of the choroidal vessels as extracted from the RGB color fundus image. The processing section 208 stores and retains the matched position in the second registration processing as the second position in the RAM 16B.

De-noise processing is not performed in the first registration processing as described above. A sharp image is present in the fundus center region 900 with less aberration compared to in the fundus peripheral region 902, and so there is comparatively less noise. This means that there is no need to perform noise removal (de-noising) on the RGB color fundus image when the registration search area is the fundus center region 900.

Moreover, as described above, the number of feature values employed in matching when performing the first registration processing is fewer than the number of feature values employed in the second registration processing. This means that an image matching processing result with good accuracy is obtained even with fewer feature points due to the density of blood vessel inside the fundus center region 900 being higher than that in the fundus peripheral region 902, reducing the compute and shortening the processing time.

Furthermore, in cases in which the second registration processing is being performed, not only are the feature points from the retinal vessels employed, but feature points of the choroidal vessels are also employed. This is because the density of the retinal vessels in the fundus peripheral region 902 is lower than in the fundus center region 900, and the density of the choroidal vessels in the fundus peripheral region 902 is higher than in the fundus center region 900, and so there is a need to employ feature points of the choroidal vessels to achieve good accuracy in the image matching processing result, and there is a need to employ both the retinal vessels and the choroidal vessels to achieve even better accuracy in the image matching processing result.

As described above, 3 individual second feature points of the retinal vessels are extracted in the image matching of the first registration processing, and both 3 individual second feature points of the retinal vessels and 3 individual second feature points of the choroidal vessels, i.e. a total of 6 individual second feature points, are extracted in the image matching of the second registration processing, however the technology disclosed herein is not limited thereto. For example, the number of feature points as denoted by "first registration processing (retinal vessels), second registration processing (retinal vessels, choroidal vessels) may, for example, be (2, 4 (2, 2)), (4, 6 (3, 3)), or the like. Thus the total number of feature points is greater in the second registration processing than in the first registration processing.

In the second registration processing there is no limitation to the number of feature points of the retinal vessels being the same as the number of feature points of the choroidal vessels. A configuration may be adopted in which the number of one thereof is greater than the number of the other thereof. For example, the number of feature points of the choroidal vessels may be made greater than the number of feature points of the retinal vessels.

The number of feature points of the choroidal vessels alone may be made greater when performing the second registration processing than the number of feature values of the retinal vessels when performing the first registration processing.

At step 362, the image processing section 206 computes an amount of eye movement from the first position to the second position. More specifically, the image processing section 206 computes a magnitude of shift and shift direction between the first position and the second position. The image processing section 206 then computes a movement amount of the examined eye 12 from the computed magnitude of shift and shift direction. This movement amount is a quantity having both a magnitude of movement and a direction of movement, i.e. is a vector quantity. Time has elapsed from the point in time when the fundus of the examined eye 12 was imaged to acquire the UWF-SLO fundus image at step 302 to the point in time when the fundus of the examined eye 12 was imaged to acquire the rectangular SLO fundus image at step 352, and so there is no certainty that the examined eye is being imaged at the same place. Moreover, sometimes the examined eye moves due to involuntary eye movements or the like during OCT imaging. At the start of OCT imaging or during OCT imaging, the image processing section 206 accordingly computes whether the examined eye 12 has shifted and in which direction and by how much, namely computes a movement amount (vector quantity) of the examined eye 12.

Note that the shift amount and shift direction that are computed at step 362 from execution at step 358 are examples of a "first shift amount and a first shift direction". The shift amount and shift direction that are computed at step 362 from execution at step 360 are examples of a "second shift amount and a second shift direction".

The examined eye 12 is shifted in this manner and examined eye 12 is scanned with reference to the position to acquire the tomographic image that was set based on the UWF-SLO fundus image, and so this results in scanning at a position shifted by this movement amount (shift amount and shift direction) from the position where acquisition would normally be desired.

At step 364, based on the movement amount (vector quantity) of the examined eye 12, the imaging control section 202 adjusts the scan range by the second optical scanner 24 such that the tomographic image can be acquired at the position for the above acquisition of the examined eye 12 after movement.

The second optical scanner 24 is an example of a "scanning device" of technology disclosed herein.

When adjustment of the scan range of the examined eye 12 has been completed in this manner, the eye-tracking processing of step 306 of Fig. 4 is ended, and the image processing proceeds to step 308.

At step 308 the imaging control section 202 controls the OCT unit 20 and the second optical scanner 24 for which the scan range has been adjusted, and acquires a tomographic image by scanning at the position of the examined eye 12 for acquiring the tomographic image.

At step 310, the processing section 208 associates the acquired tomographic image, the RG-UWF-SLO fundus image, and the data of the position 402 for acquiring the tomographic image with the examinee information, and outputs these to the server 140. This completes the image processing of Fig. 4.

The server 140 stores the tomographic image, the RGB color fundus image, and data of the position 402 for acquiring the tomographic image in a non-illustrated storage device associated with the examinee ID.

A request from the viewer 150 to transmit data such as a tomographic image corresponding to the examinee ID is given to the server 140 by instruction from the user, such as an ophthalmologist.

When the server 140 has been given such a request, the server 140 transmits the tomographic image, the UWF-SLO fundus image, and the data of the position 402 for acquiring the tomographic image that were stored associated with the examinee ID to the viewer 150, associated with the examinee information and the like. The viewer 150 thereby displays the respective data received on a non-illustrated display.

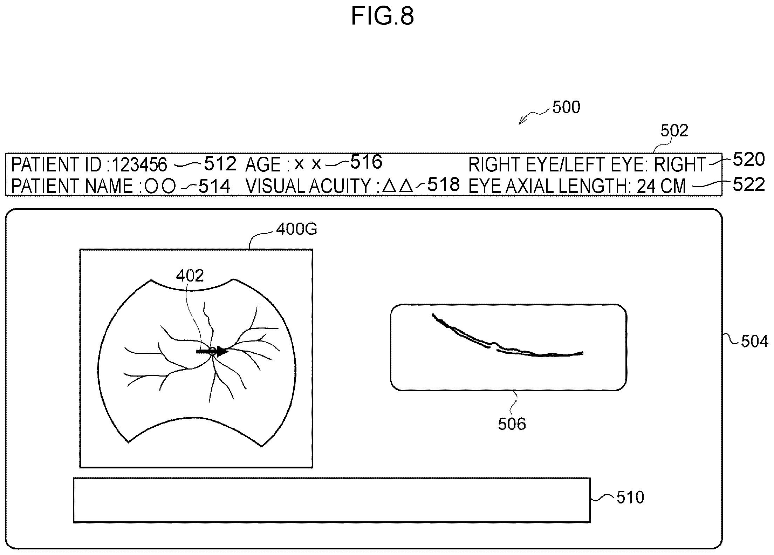

Fig. 8 illustrates a screen 500 displayed on the display of the viewer 150. The screen 500 includes an examinee information display area 502 and an image display area 504.

The examinee information display area 502 includes an examinee ID display field 512, an examinee name display field 514, an age display field 516, a visual acuity display field 518, a right eye/left eye display field 520, and an eye axial length display field 522. The display control section 204 displays the examinee ID, the examinee name, the examinee age, visual acuity, right eye/left eye information, and the examinee eye axial length data that have been received from the server 140 in the corresponding display fields 512 to 522.

The image display area 504 includes an RGB color fundus image display field 508, a tomographic image display field 506, and a text data display field 510.

The UWF-SLO fundus image 400G is displayed in the RGB color fundus image display field 508 with the position 402 for acquiring the tomographic image superimposed thereon.

A tomographic image is displayed in the tomographic image display field 506. Diagnostic comments and the like are displayed in the text data display field 510.

As described above, in the present exemplary embodiment the first registration processing is executed when at least part of the region 400 for acquiring the rectangular SLO fundus image is contained in the specific range of the UWF-SLO fundus image. More specifically, the shift amount and shift direction of the examined eye 12 is computed using a comparatively small number of feature points of the retinal vessels respectively in the UWF-SLO fundus image and the rectangular SLO fundus image. This thereby enables the shift amount and shift direction of the examined eye 12 to be computed in a comparatively short period of time. The movement amount of the examined eye 12 can accordingly be computed in a comparatively short period of time. The scan range for the second optical scanner 24 is accordingly adjusted based on the movement amount of the examined eye 12 as computed in the comparatively short period of time, and the tomographic image is acquired. Thus in the above exemplary embodiment a tomographic image is accordingly able to be acquired while tracking eye movement.

In the present exemplary embodiment the second registration processing is executed when not even part of the region 400 for acquiring the rectangular SLO fundus image is contained in the specific range of the UWF-SLO fundus image. More specifically, the shift amount and shift direction of the examined eye 12 are computed using feature points respectively in the UWF-SLO fundus image and the rectangular SLO fundus image, with there being comparatively more feature points than previously for at least the choroidal vessels. This accordingly enables the shift amount and shift direction of the examined eye 12 to be computed accurately. This accordingly enables the movement amount of the examined eye 12 to be computed accurately. The scan range by the second optical scanner 24 is accordingly adjusted based on the accurately computed movement amount of the examined eye 12, and the tomographic image is acquired. The exemplary embodiment described above is accordingly able to accurately acquire a tomographic image of a set region.

In the exemplary embodiment described above, the tomographic image is acquired after the eye-tracking processing has been executed. However the technology disclosed herein is not limited thereto. For example, the eye-tracking processing may be executed while the tomographic image is being acquired. In such cases, a configuration may be adopted in which the processing from step 352 to step 362 of Fig. 5 is executed repeatedly while the tomographic image is being acquired. A configuration may be adopted in which a mean value is found of the movement amounts of the examined eye 12 obtained each time the processing from step 352 to step 362 is repeatedly executed a specific number of times, and the scan range by the second optical scanner 24 is adjusted while the tomographic images are being acquired so as to acquire the tomographic images. Adopting such an approach means that plural frames of tomographic image are acquired at the same position of the examined eye 12. A tomographic image with reduced noise may be acquired by obtaining the arithmetic mean of these plural frames of tomographic image.

In the exemplary embodiment described above the tomographic image is acquired after the eye-tracking processing has been executed. The technology disclosed herein is not limited thereto. For example, plural frames of tomographic image may be acquired without tracking the optical scanner. A configuration may be adopted in which the processing from step 352 to step 362 of Fig. 5 is repeatedly executed while the plural frames of tomographic image are being acquired, then any tomographic image that were imaged when eye movement was a specific value or greater are removed, and an arithmetic mean obtained using the remaining plural frames of tomographic image.

There may be one of each of the configuration elements (devices or the like), or two or more thereof, provided in the present disclosure unless this results in a contradiction.

Although explanation has been given in the examples described above regarding examples in which image processing is implemented using a software configuration utilizing a computer, the technology disclosed herein is not limited thereto. For example, instead of the software configuration utilizing a computer, the image processing may be executed solely by a hardware configuration such as a field programmable gate array (FPGA) or an application specific integrated circuit (ASIC). Alternatively, a configuration may be adopted in which some processing out of the image processing is executed by a software configuration, and the remaining processing is executed by a hardware configuration.

Thus technology disclosed herein includes cases in which the image processing is implemented by a software configuration utilizing a computer and cases in which the image processing is not implemented thereby, and so encompasses the following technology.

### First Technology

An image processing device including:
a first acquisition section that acquires a first fundus image of an examined eye;
a second acquisition section that acquires a position for acquiring a tomographic image of the examined eye fundus set using the first fundus image;
a third acquisition section that acquires a second fundus image of the examined eye;
a determination section that determines whether or not the acquired position is contained in a specific range of the first fundus image; and
a computation section that computes a first shift amount and shift direction of the examined eye when the acquired position is contained in the specific range by computation using first registration processing to positionally align the first fundus image and the second fundus image, and that computes a second shift amount and shift direction of the examined eye when the acquired position falls outside the specific range by computation using second registration processing to positionally align the first fundus image and the second fundus image different to the first registration processing.

### Second Technology

An image processing method including:
a step of a first acquisition section acquiring a first fundus image of an examined eye;
a step of a second acquisition section acquiring a position for acquiring a tomographic image of the examined eye fundus set using the first fundus image;
a step of a third acquisition section acquiring a second fundus image of the examined eye;
a step of a determination section determining whether or not the acquired position is contained in a specific range of the first fundus image; and
a step of a computation section computing a first shift amount and shift direction of the examined eye when the acquired position is contained in the specific range by computation using first registration processing to positionally align the first fundus image and the second fundus image, and computing a second shift amount and shift direction of the examined eye when the acquired position falls outside the specific range by computation using second registration processing to positionally align the first fundus image and the second fundus image different to the first registration processing.

The imaging control section 202 is an example of a "first acquisition section" and a "third acquisition section" of technology disclosed herein. The processing section 208 is an example of a "second acquisition section" of technology disclosed herein. The image processing section 206 is an example of a "determination section" and a "computation section" of technology disclosed herein.

The following technology is proposed from the content disclosed above.

### Third Technology

A computer program product for image processing, the computer program product including a computer-readable storage medium that is not itself a transitory signal and a program stored on the computer-readable storage medium, and the program causes image processing to be executed by a processor on a computer. The image processing includes a step of acquiring a first fundus image of an examined eye, a step of acquiring a position for acquiring a tomographic image of the examined eye fundus set using the first fundus image, a step of acquiring a second fundus image of the examined eye, a step of determining whether or not the acquired position is contained in a specific range of the first fundus image, and a step of computing a first shift amount and shift direction of the examined eye when the acquired position is contained in the specific range by computation using first registration processing to positionally align the first fundus image and the second fundus image, and computing a second shift amount and shift direction of the examined eye when the acquired position falls outside the specific range by computation using second registration processing to positionally align the first fundus image and the second fundus image different to the first registration processing.

It must be understood that the respective image processing described above are merely examples thereof. Obviously redundant steps may be omitted, new steps may be added, and the processing sequence may be swapped around within a range not departing from the spirit of the technology disclosed herein.

All publications, patent applications and technical standards mentioned in the present specification are incorporated by reference in the present specification to the same extent as if each individual publication, patent application, or technical standard was specifically and individually indicated to be incorporated by reference.

## Claims

1. An image processing method for image processing performed by a processor, the image processing comprising:
a step of acquiring a first fundus image of an examined eye;
a step of acquiring a position for acquiring a tomographic image of the examined eye's fundus, which is set using the first fundus image;
a step of acquiring a second fundus image of the examined eye;
a step of determining whether or not the acquired position is included in a specific range of the first fundus image; and
a step of computing a first movement amount of the examined eye in a case in which the acquired position is included in the specific range, using first registration processing to positionally align the first fundus image and the second fundus image, and of computing a second movement amount of the examined eye in a case in which the acquired position falls outside the specific range, using second registration processing to positionally align the first fundus image and the second fundus image, the second registration processing being different from the first registration processing.

2. The image processing method of claim 1, further comprising a step of controlling a scanning device for acquiring the tomographic image, based on the first movement amount or on the second movement amount.

3. The image processing method of either claim 1 or claim 2, wherein the specific range is a fundus center region.

4. The image processing method of any one from claim 1 to claim 3, further comprising a step of extracting a feature point of respective retinal vessels from the first fundus image and the second fundus image,
wherein the first registration processing positionally aligns the first fundus image and the second fundus image using the feature points of the retinal vessels.

5. The image processing method of any one from claim 1 to claim 4, further comprising a step of extracting a feature point of respective choroidal vessels from the first fundus image and the second fundus image,
wherein the second registration processing positionally aligns the first fundus image and the second fundus image using the feature points of the choroidal vessels.

6. The image processing method of any one from claim 1 to claim 5, wherein:
the first movement amount is configured by components comprising a first shift amount and a first shift direction; and
the second movement amount is configured by components comprising a second shift amount and a second shift direction.

7. The image processing method of any one of claim 1 to claim 6 wherein:
the first fundus image includes an image of the examined eye's fundus itself formed by guiding light reflected from the examined eye's fundus itself; and
the step of acquiring the second fundus image of the examined eye includes using the first fundus image to acquire a position of a region that is configured so as to contain at least a part of an image of the examined eye's fundus itself, and acquiring the second fundus image by acquiring an image of the examined eye's fundus based on the position of the region acquired.

8. The image processing method of claim 7, wherein the size of the region is smaller than the size of the first fundus image.

9. The image processing method of claim 7 or claim 8, wherein the region includes at least a part of the position for acquiring the tomographic image.

10. The image processing method of claim 2, further comprising a step of using the controlled scanning device to acquire a tomographic image of the examined eye's fundus.

11. The image processing method of claim 2, further comprising a step of acquiring a tomographic image of the examined eye fundus while repeatedly executing the step of acquiring the second fundus image, the step of determining, the step of computing, and the step of controlling.

12. An image processing device comprising a processor, wherein the processor executes image processing comprising:
a step of acquiring a first fundus image of an examined eye;
a step of acquiring a position for acquiring a tomographic image of the examined eye's fundus, which is set using the first fundus image;
a step of acquiring a second fundus image of the examined eye;
a step of determining whether or not the acquired position is included in a specific range of the first fundus image; and
a step of computing a first movement amount of the examined eye in a case in which the acquired position is included in the specific range, using first registration processing to positionally align the first fundus image and the second fundus image, and of computing a second movement amount of the examined eye in a case in which the acquired position falls outside the specific range, using second registration processing to positionally align the first fundus image and the second fundus image, the second registration processing being different from the first registration processing.

13. A program that causes a computer to execute image processing, the image processing comprising:
a step of acquiring a first fundus image of an examined eye;
a step of acquiring a position for acquiring a tomographic image of the examined eye's fundus, which is set using the first fundus image;
a step of acquiring a second fundus image of the examined eye;
a step of determining whether or not the acquired position is included in a specific range of the first fundus image; and
a step of computing a first movement amount of the examined eye in a case in which the acquired position is included in the specific range, using first registration processing to positionally align the first fundus image and the second fundus image, and of computing a second movement amount of the examined eye in a case in which the acquired position falls outside the specific range, using second registration processing to positionally align the first fundus image and the second fundus image, the second registration processing being different from the first registration processing.
